(19) Europäisches Patentamt

European Patent Office

Office européen des brevets

(11) EP 1 563 829 A1

(12) **DEMANDE DE BREVET EUROPEEN**

(43) Date de publication:
17.08.2005 Bulletin 2005/33

(51) Int Cl.7: **A61K 7/32**, A61K 7/48

(21) Numéro de dépôt: 05290136.0

(22) Date de dépôt: 21.01.2005

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **10.02.2004 FR 0450230**

(71) Demandeur: **L'OREAL**
**75008 Paris (FR)**

(72) Inventeurs:
• **Lemoine, Cyril**
**95380 Puiseaux-en-France (FR)**
• **Beau, Nathalie**
**95610 Eragny-sur-Oise (FR)**
• **Prud' Homme, Estelle**
**75018 (FR)**

(74) Mandataire: **Miszputen, Laurent**
**L'OREAL - D.I.P.I.**
**25-29 Quai Aulagnier**
**92600 Asnières (FR)**

(54) **Composition de gluconate de zinc comprenant des particules sous forme aciculaire**

(57) L'invention concerne de nouvelles compositions de particules de gluconate de zinc comprenant au moins des particules de gluconate de zinc sous forme aciculaire et leurs procédés de préparation.

L'invention a pour objet une formulation cosmétique déodorante comprenant dans un support cosmétiquement acceptable au moins une composition de particules de gluconate de zinc contenant au moins des particules de gluconate de zinc sous forme aciculaire.

L'invention concerne aussi l'utilisation d'une composition de particules de gluconate de zinc contenant au moins des particules sous forme aciculaire comme actif déodorant dans une composition cosmétique.

L'invention concerne également un procédé de traitement cosmétique de la transpiration humaine et des odeurs axillaires humaines à l'aide de cette composition.

Printed by Jouve, 75001 PARIS (FR)

**Description**

L'invention concerne de nouvelles compositions de particules de gluconate de zinc comprenant au moins des particules de gluconate de zinc sous forme aciculaire et leurs procédés de préparation.

L'invention a pour objet une formulation cosmétique déodorante comprenant dans un support cosmétiquement acceptable au moins une composition de particules de gluconate de zinc contenant au moins des particules de gluconate de zinc sous forme aciculaire.

L'invention concerne également un procédé de traitement cosmétique de la transpiration humaine et des odeurs axillaires humaines à l'aide de cette composition.

Dans le domaine cosmétique, il est bien connu d'utiliser en application topique, des produits déodorants contenant des substances actives de type anti-transpirant, de type bactéricide ou du type absorbeur d'odeurs pour diminuer, voire supprimer, les odeurs axillaires généralement désagréables.

Les substances bactéricides inhibent le développement de la flore cutanée responsable des odeurs axillaires. Ils ont pour inconvénient de ne pas être actifs sur l'odeur de la sueur déjà développée. Parmi les produits bactéricides, le plus couramment employé est le Triclosan (2,4,4'-trichloro-2'-hydroxydiphényléther), qui présente l'inconvénient de modifier de façon importante l'écologie de la flore cutanée, d'être inhibé par certains composés, comme par exemple les tensio-actifs non-ioniques, couramment utilisés dans la formulation de compositions cosmétiques. Par ailleurs, le caractère insoluble du Triclosan dans l'eau ne permet pas son incorporation dans des formules essentiellement aqueuses.

Les substances anti-transpirantes ont pour effet de limiter le flux sudoral. Elles sont généralement constituées de sels d'aluminium. Leur efficacité déodorante est limitée lorsqu'ils sont utilisés seuls. De plus, dans des concentrations élevées, ces susbstances présentent un potentiel irritant pour la peau.

On connaît dans le brevet EP768080 des compositions déodorantes aqueuses en particulier des émulsions eau/silicone contenant comme actif absorbeur d'odeurs des sels de zinc hydrosolubles comme le gluconate de zinc.

On connaît également dans la demande de brevet WO 01/99376 des compositions déodorantes contenant des inhibiteurs d'arylsulfatase parmi lesquels sont mentionnés les sels d'aluminium et gluconate de zinc. Ce document décrit en particulier un exemple de stick anti-transpirant contenant 20% en poids d'aluminium chlorhydrate et 0,1% de gluconate de zinc. Ce type de composition produit un taux élevé de résidu blanc sur la peau après application.

Les gluconates de zinc utilisés dans l'art antérieur sont soit sous forme dissoute dans des émulsions telles que des émulsions eau/silicone soit sous forme de particules en suspension dont la taille moyenne est généralement inférieure à 170 µm. Ces particules ne se présentent pas sous forme d'aiguilles. Leur efficacité n'est pas pleinement satisfaisante. Les gluconates de zinc de l'art antérieur sont difficilement formulables dans les supports dans lesquels ceux ci sont en suspension notamment dans les sticks anhydres, les aérosols anhydres ou alcooliques. Les particules de gluconate de zinc classiques ont tendance à décanter et conduisent à la délivrance de produits hétérogènes. D'autre part, dans le cadre des aérosols, au bout de quelques jours de stockage, le gâteau de sédimentation des particules de gluconate de zinc classiques qui se forme au fond du récipient est souvent difficile à redisperser après agitation voire reste coller au fond.

La demanderesse a découvert de manière surprenante une nouvelle composition de particules de gluconate de zinc comprenant au moins des particules de gluconate de zinc sous forme aciculaire à savoir sous forme d'aiguilles présentant une efficacité déodorante supérieure à celle des particules de gluconate de zinc classiques. Elle a également découvert que les compositions de gluconate de zinc de l'invention pouvaient être facilement formulées en suspension dans des compositions déodorantes et en particulier dans les sticks anhydres et dans les aérosols anhydres ou alcooliques sans les inconvénients énumérés précédemment.

De façon plus précise, l'invention a pour objet une composition de particules de gluconate de zinc comprenant au moins des particules de gluconate de zinc sous forme aciculaire.

L'invention a également pour objet divers procédés de préparation d'une composition de particules de gluconate de zinc comprenant au moins des particules de gluconate de zinc sous forme aciculaire.

On entend par "particules sous forme aciculaire" des particules sous forme d'aiguilles.

L'invention a pour objet une formulation cosmétique déodorante comprenant dans un support cosmétiquement acceptable au moins une composition de gluconate de zinc comprenant au moins des particules de gluconate de zinc sous forme aciculaire.

Au sens de la présente invention, on entend par "composition déodorante" toute composition capable de réduire le flux sudoral, masquer, absorber, améliorer et/ou réduire l'odeur désagréable résultant de la décomposition de la sueur humaine par des bactéries.

L'invention concerne également un procédé de traitement cosmétique de la transpiration humaine et des odeurs axillaires humaines à l'aide de cette composition.

La composition de gluconate de zinc conforme à l'invention peut être constituée d'un mélange de particules de gluconate de zinc non-aciculaire et de particules de gluconate de zinc sous forme aciculaire.

Selon une forme particulière de l'invention, la composition de gluconate de zinc conforme à l'invention peut être constituée exclusivement de particules de gluconate de zinc sous forme aciculaire.

Les particules de gluconate de zinc sous forme aciculaire conformes à l'invention ont de préférence une longueur moyenne allant de 1 à 200 µm et un diamètre inférieur à 5µm. Plus particulièrement, leur longueur moyenne variera de 5 à 50 µm et un diamètre sera inférieur à 1 µm.

Les particules de gluconate de zinc sous forme non-aciculaire ont en général une taille de particule moyenne inférieure à 200 µm et de préférence allant de 5 à 165 µm et plus préférentiellement ayant une granulométrie moyenne pondérée en volume D(4,3) d'environ de 70 µm.

La composition de particules de gluconate de zinc conforme à l'invention peut être également sous forme d'une suspension dans un mélange de solvants comprenant au moins un solvant (A) solubilisant le gluconate de zinc et au moins un solvant (B) non-solubilisant le gluconate de zinc ; les proportions entre les solvants (A) et (B) étant telles que le gluconate de zinc est non-soluble dans le mélange de solvants (A) et (B) à température ambiante.

On entend par mélanges ou solvants « solubilisants » le gluconate de zinc, tout milieu qui permet la solubilisation d'au moins 1% en poids de gluconate de zinc à température ambiante.

A titre d'exemples de solvants (A) solubilisant le gluconate de zinc, on peut citer l'eau et la glycérine ou leurs mélanges.

A titre d'exemples de solvants (B) ne solubilisant pas le gluconate de zinc, on peut citer les monoalcools à chaîne courte par exemple en $C_1$-$C_4$ comme l'éthanol , l'isopropanol ; les mono- ou polyalkylène glycols comme l'éthylène glycol, le 1,2-propylène glycol, le 1,3-butylène glycol, 1,2-pentanediol, l'hexylène glycol, le diéthylène glycol, le dipropylene glycol ou leurs éthers; ainsi que leurs mélanges.

La composition de gluconate de zinc selon l'invention peut être obtenue selon un premier procédé de préparation comprenant les étapes suivantes

(a) un gluconate de zinc sous forme de poudre non aciculaire est mis en contact sous forte agitation avec au moins un solvant (A) solubilisant du gluconate de zinc et au moins un solvant (B) non-solubilisant dudit gluconate de zinc ; les proportions entre les solvants (A) et (B) étant telles que le gluconate de zinc est non-soluble dans le mélange de solvants (A) et (B).

(b) le mélange résultant est mis sous agitation jusqu'à formation de cristaux de gluconate de zinc en aiguilles et peut être chauffé jusqu'à 50°C pour accélérer la transformation du gluconate de zinc en particules aciculaires

(c) éventuellement, après formation des aiguilles, on effectue une élimination des solvants par filtration ou évaporation.

Cette cristallisation aciculaire est visible par microscopie.

Un autre objet de l'invention consiste en une composition de particules de gluconate de zinc susceptible d'être obtenue par ledit procédé de préparation.

La composition de particules de gluconate selon l'invention peut être obtenue selon un deuxième procédé de préparation comprenant les étapes suivantes

(a) on solubilise un gluconate de zinc sous forme de poudre non aciculaire dans au moins un solvant (A) solubilisant ledit gluconate de zinc ;

(b) on chauffe la solution à une température T supérieure ou égale à 40 °C;

(c) on introduit au moins un solvant (B) ne solubilisant pas le gluconate de zinc ; les proportions entre les solvants (A) et (B) étant telles que le gluconate de zinc est soluble à la température T dans le mélange de solvants (A) et (B) et insoluble dans le même mélange à température ambiante ;

(d) le mélange résultant est refroidi à une température inférieure ou égale à la température ambiante sous agitation,

(e) éventuellement, après formation des aiguilles, on effectue ensuite une élimination des solvants par filtration ou évaporation.

Un autre objet de l'invention consiste en une composition de particules de gluconate de zinc susceptible d'être obtenue par ledit procédé de préparation.

Les particules de gluconate de zinc obtenues selon ces procédés peuvent se trouver sous forme de mélange de gluconate de zinc non aciculaire et de gluconate de zinc aciculaire (la transformation de la forme classique en forme aciculaire n'est pas complète).

Les particules de gluconate de zinc obtenues selon ces procédés peuvent se trouver sous forme aciculaire pure (la transformation de la forme classique en forme aciculaire est complète).

L'invention a pour objet une formulation cosmétique déodorante comprenant dans un support cosmétiquement acceptable au moins une composition de gluconate de zinc comprenant au moins des particules de gluconate de zinc sous forme aciculaire.

La composition de particules de gluconate de zinc selon l'invention est de préférence présente dans les formulations déodorantes à raison d'environ 0,01 à 10% en poids et plus préférentiellement de 0,1 à 5% en poids par rapport au poids total de la composition.

Les compositions déodorantes selon l'invention sont destinées à l'usage cosmétique et peuvent se présenter sous forme de lotions, de crèmes ou de gels fluides distribués en spray aérosol, en flacon pompe ou en roll-on, sous forme de crèmes épaisses distribuées en tubes ou en grille ; sous forme de bâtonnets (sticks), et contenir à cet égard les ingrédients généralement utilisés dans ce type de produits et bien connus de l'homme de l'art, sous réserve qu'ils n'interfèrent pas avec le gluconate de zinc décrit dans la présente invention.

Les compositions déodorantes selon l'invention destinées à l'usage cosmétique peuvent comporter au moins une phase aqueuse. Elle peuvent être notamment formulées sous forme de lotions ou sous forme d'émulsion comme une émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse desdites compositions contient de l'eau et en général d'autres solvants solubles ou miscibles dans l'eau qui ne permettent pas de solubiliser les particules de gluconate de zinc sous forme aciculaire. Ils peuvent être choisis par exemple parmi les solvants (B) tels que cités précédemment ou leurs mélanges avec des solvants (A) tels que cités précédemment. Dans tous les cas, cette phase aqueuse ne permet pas de solubiliser le gluconate de zinc.

Selon une forme particulière de l'invention, les compositions selon l'invention peuvent être anhydres.

Par "anhydre", on entend au sens de l'invention, une composition dont la teneur en eau libre ou ajoutée est inférieure à 3% et de préférence dont la teneur en eau ajoutée est inférieure à 1 % en poids par rapport au poids total de la composition.

Les compositions selon l'invention peuvent contenir une phase liquide organique non-miscible dans l'eau. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones $D_4$, $D_5$ ou $D_6$.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les huiles émollientes non-volatiles utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{18}$ et leurs mélanges, des polyols en $C_2$-$C_6$ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

Les formulations selon l'invention peuvent également contenir également un ou plusieurs autres agents structurants ou gélifiants de la phase liquide organique non-miscible dans l'eau de la formulation tels que les alcools gras solides linéaires et/ou les cires ; les acides gras ou leurs sels (acide stéarique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ; les organopolysiloxane élastomères tels que ceux décrits dans la demande WO97/44010.

La formulation cosmétique déodorante selon l'invention peut contenir un ou plusieurs actifs déodorants additionnels.

Parmi les actifs déodorants additionnels, on peut citer les sels d'aluminium anti-transpirants.

Par " sel d'aluminium anti-transpirant" , on entend tout sel ou tout complexe d'aluminium ayant pour effet de diminuer ou limiter le flux sudoral.

Les sels d'aluminium conformes à l'invention sont choisis de préférence parmi les halohydrates de d'aluminium ; les halohydrates d'aluminium et de zirconium, les complexes d'hydroxychlorure de zirconium et d'hydroxychlorure d'aluminium avec un acide aminé tels que ceux décrits dans le brevet US-3792068 communément connus sous l'appellation "ZAG complexes".

Parmi les sels d'aluminium, on peut citer en particulier le chlorhydrate d'aluminium sous forme activée ou non activée, l'aluminium chlorhydrex, le complexe aluminium chlorhydrex polyéthylèneglycol, le complexe aluminium chlorhydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Les sels d'aluminium anti-transpirants peuvent être présents dans la composition selon l'invention à raison d'environ 0,5 à 25% en poids par rapport au poids total de la composition.

Parmi les actifs déodorants additionnels, on peut également citer des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Afin d'améliorer l'homogénéité du produit, on peut utiliser, en plus, un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une charge.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans les compositions déodorantes.

Selon une forme particulièrement préféré de l'invention, les formulations déodorantes se présentent sous forme aérosol alcoolique à savoir qu'elles contiennent en plus du gluconate au moins un mono-alcool en $C_1$-$C_4$ et au moins un agent propulseur.

Les formulations selon l'invention peuvent être pressurisées et être conditionnées dans un dispositif aérosol.

La présente invention a également pour objet un dispositif aérosol constitué par :

(A) un récipient comprenant une formulation déodorante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

La présente invention a également pour objet un procédé cosmétique pour traiter les odeurs axillaires humaines, consistant à appliquer sur la surface axillaire une quantité efficace d'une composition de particules de gluconate de zinc telle que définie précédemment ou d'une formulation déodorante la contenant telle que définie précédemment.

La présente invention a également pour objet l'utilisation d'une composition de particules de gluconate de zinc telle que définie précédemment comme actif déodorant dans une composition cosmétique.

Les exemples qui suivent servent à illustrer la présente invention.

**EXEMPLES DE PROCEDE :**

**EXEMPLES 1A, 2A ET 3A DE PROCEDE**

On prépare les compositions de particules de gluconate de zinc 1A, 2A et 3A selon le mode opératoire suivant :
Les différents solvants sont mélangés dans un récipient jusqu'à homogénéisation du milieu. Le gluconate de zinc est ajouté au mélange sous agitation avec pales et raclantes à température ambiante (23°C). Les temps d'agitation des préparations 1A ; 2A et 3A sont respectivement 24heures ; 6 heures et 12 heures. Le mélange est dispersé au turax à une vitesse de 10 000 tours /minutes pendant 5 minutes en fin de préparation.

Les quantités sont exprimées en pourcentages en poids.

| Ingrédients | 1A | 2A | 3A |
|---|---|---|---|
| Gluconate de zinc non-aciculaire (Givobio G Zn- Seppic) | 2 | 2 | 1 |
| Ethanol absolu | 93 | 58 | 83,5 |
| Eau | 5 | 40 | 4,4 |
| Di Propylene Glycol | / | / | 11,1 |
| | | | |
| Temps d'agitation (heures) | 24 | 6 | 12 |

Les particules de gluconate se trouvent en partie sous leur forme d'origine et en partie sous forme d'aiguilles. Les caractéristiques granulométriques des particules de gluconate de zinc aciculaires des préparations ci-dessus sont :

| Longueur des aiguilles | 1A | 2A | 3A |
|---|---|---|---|
| Inférieur à 10 $\mu$m | 37% | 20% | 70% |
| Inférieur à 20 $\mu$m | 81% | 56% | 98% |
| Inférieur à 30 $\mu$m | 99% | 71% | |
| Inférieur à 50 $\mu$m | | 86% | |
| Inférieur à 100 $\mu$m | | 97% | |
| Moyenne ($\mu$m) | 13 | 28 | 9 |

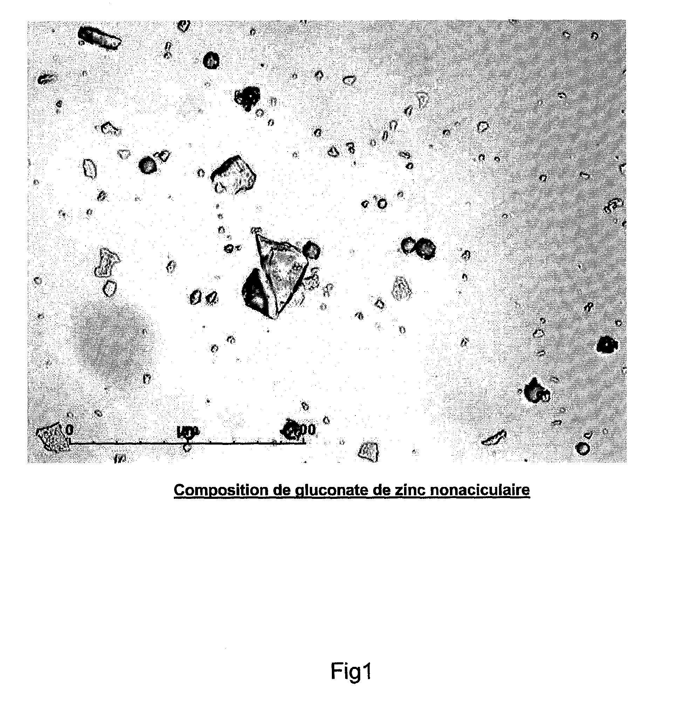

Les particules de gluconate de zinc non-aciculaire d'origine ont été observées au microscope et photographiées. La photographie est représentée en figure 1.

## EXEMPLES 1B, 2B ET 3B DE PROCEDE

On prépare les compositions de particules de gluconate de zinc 1 B, 2B suivantes selon le mode opératoire suivant :

Le gluconate de zinc est solubilisé dans l'eau à 60°C jusqu'à obtention d'une solution transparente. L'éthanol est ajouté lentement à la solution tout en gardant le mélange homogène et transparent à 60°C. Le mélange est alors refroidi sous agitation avec pale et raclante jusqu'à température ambiante. La cristallisation du gluconate de zinc à lieu lors du refroidissement vers 40-50°C. Le gluconate de zinc se trouve alors presque totalement sous forme aciculaire.

Les quantités sont exprimées en pourcentages en poids.

| Ingrédients | 1B | 2B | 3B |
|---|---|---|---|
| Gluconate de zinc non-aciculaire (Givobio G Zn- Seppic) | 6,8 | 16,7 | 15,4 |
| Eau | 48 | 66,6 | 53,8 |
| Ethanol absolu | 45,2 | 16,7 | 30,8 |

Les compositions de gluconate de zinc sont presque totalement constituées de particules sous forme aciculaire avec une granulométrie telle que :

| Longueur des aiguilles | 1B | 2B | 3B |
|---|---|---|---|
| Inférieur à 10 $\mu$m | 21% | 20% | 43% |
| Inférieur à 20 $\mu$m | 62% | 72% | 96% |

(suite)

| Longueur des aiguilles | 1B | 2B | 3B |
|---|---|---|---|
| Inférieur à 30 μm | 88% | 95% | 99% |
| Inférieur à 50 μm | 97% | 99% | |
| Moyenne (μm) | 19 | 17 | 12 |

Les compositions de gluconate de zinc ainsi obtenues ont été observées au microscope et photographiées.

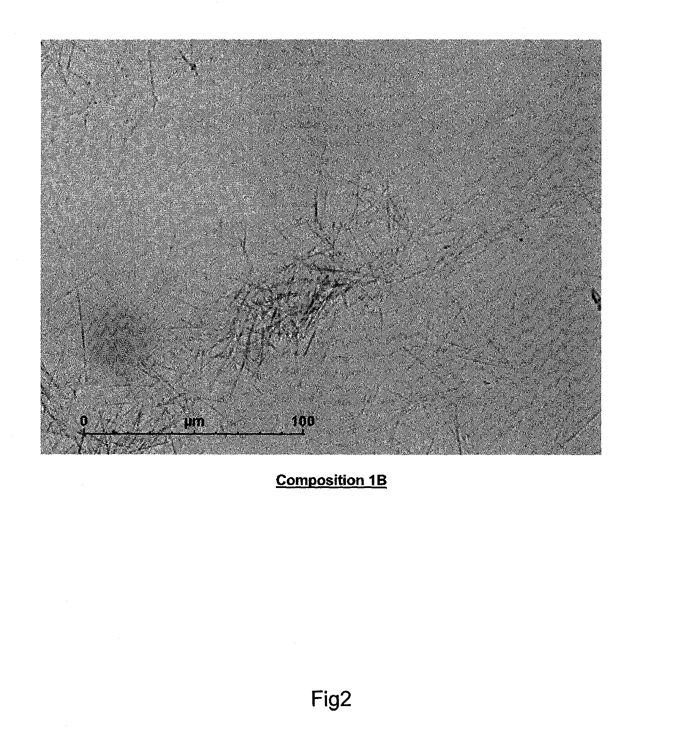

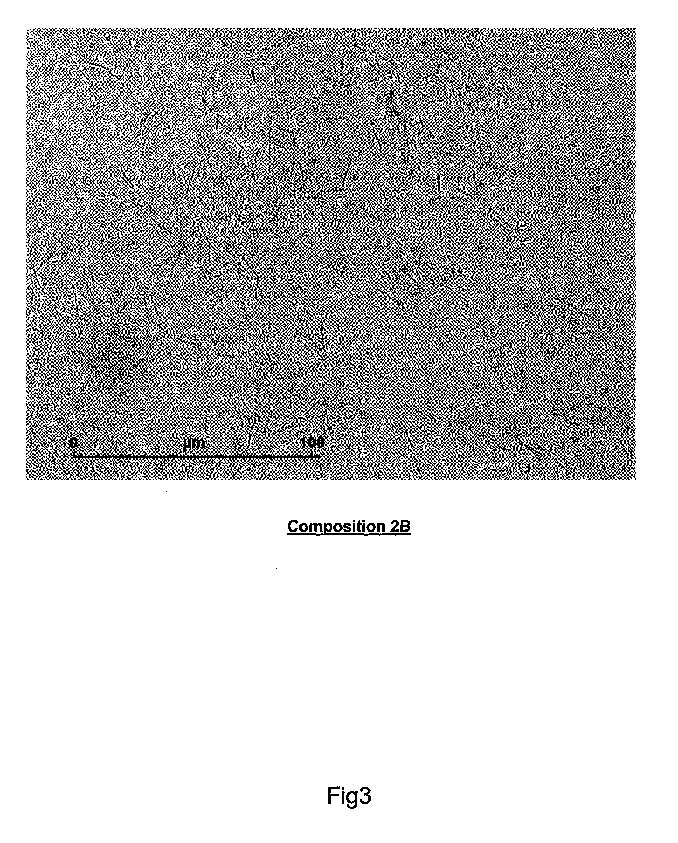

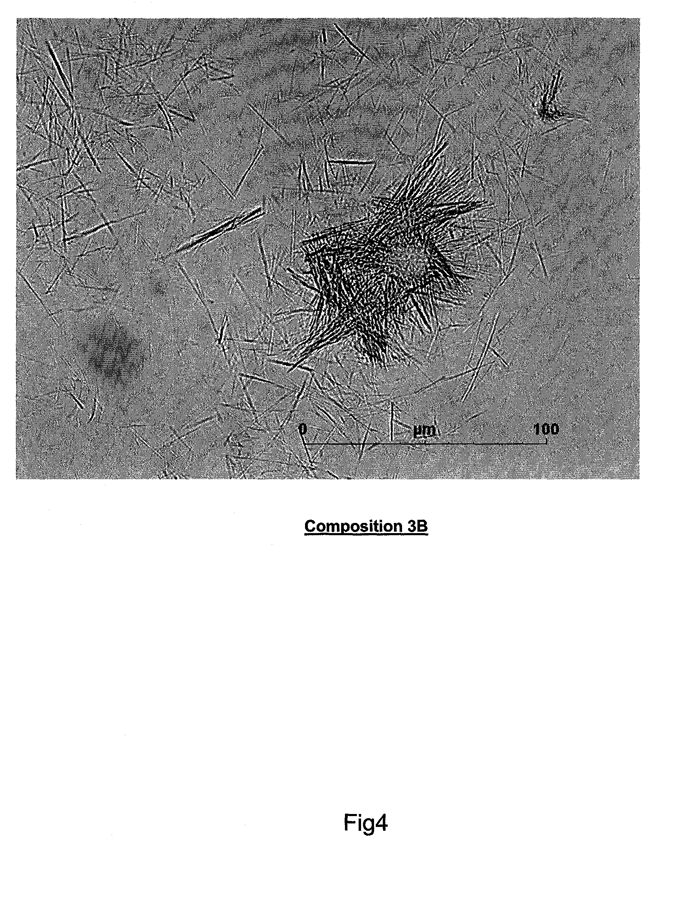

La photographie de la composition 1 B est représentée en figure 2.
La photographie de la composition 2B est représentée en figure 3.
La photographie de la composition 3B est représentée en figure 4.

**ETUDE D'EFFICACITE COMPARATIVE IN-VITRO D'UNE COMPOSITION DE PARTICULES DE GLUCONATE DE ZINC SELON L'INVENTION PAR RAPPORT A UN GLUCONATE DE ZINC NON-ACICULAIRE:**

La composition de particules de gluconate selon l'invention est préparée suivant l'exemple 1A puis filtrée.

Des recueils de sueur axillaire sont effectués en sauna sur 6 volontaires, les échantillons de sueur individuelle, conservés dans la glace pendant quelques heures, sont alors pratiquement inodores. Ils sont ensuite mélangés et répartis en aliquots de 1 ml. Les actifs sont rajoutés à ces aliquots, puis mis à incuber à l'étuve 37°C. Après 18 et 24 heures d'incubation, un jury d'experts évalue l'intensité de l'odeur comparativement à un échantillon témoin : 1 ml de sueur incubée sans ajout d'actif. L'intensité est évaluée sur une échelle de 0 (pas d'odeur) à 4 (très forte odeur).

Les résultats sont exprimés en % de variation de l'intensité du désagrément de l'odeur comparativement à cet échantillon de sueur témoin (moyenne des % de variation à T18h et T24h).

$$\Delta = \frac{(\text{intensité odeur de l'échantillon témoin - intensité odeur de l'échantillon avec actif})}{\text{intensité odeur de l'échantillon témoin}} \times 100$$

| Composition testée | Concentration en mg/ml de sueur | Moyenne des % de variation de l'intensité de l'odeur |
|---|---|---|
| Composition de particules de gluconate de zinc selon l'exemple 1A (invention) | 0,5 | -68 |
| Gluconate de zinc non-aciculaire (Givobio G Zn- Seppic) (hors invention) | 0,5 | -18 |

On observe que la composition de particules de gluconate de zinc selon l'invention comprenant des particules sous forme aciculaire est plus efficace que des particules de gluconate de zinc classiques non-aciculaires.

**ETUDE COMPARATIVE DE L'ACTITIVITE DEODORANTE IN-VIVO D'UNE COMPOSITION DE GLUCONATE DE ZINC SELON L'INVENTION CONTRE LE TRICLOSAN SOUS FORME D'AEROSOL ALCOOLIQUE :**

La composition de particules de gluconate de zinc selon l'invention est préparée suivant le mode opératoire de l'exemple 1A. On réalise les aérosols alcooliques 2 et 3 suivants; les quantités sont exprimées en pourcentages en poids. Les préparations sont ensuite conditionnées en aérosol et pressurisées selon une méthode classique.

| Noms chimiques | Exemple 2 (invention) | Exemple 3 (hors invention) |
|---|---|---|
| Triclosan | | 0.15 |
| Composition de particules de gluconate de zinc selon l'exemple 1A. | 45 | |

(suite)

| Noms chimiques | Exemple 2 (invention) | Exemple 3 (hors invention) |
|---|---|---|
| Alcool éthylique à 95°vol dénaturé | | 44,85 |
| Isobutane | 55 | 55 |

**Protocole de tests :**

L'étude est réalisée contre l'aisselle non-traitée de 20 volontaires. On effectue une seule application à raison de 1,2 g ± 0.05 g vaporisé à 15 cm de l'aisselle. Chaque aérosol déodorant est appliqué après essuyage de l'aisselle. L'aisselle témoin est également essuyée mais ne reçoit aucun traitement. Les évaluations de l'intensité de l'odeur de transpiration et du désagrément (valeur hédonique) de celle-ci sont effectuées par sniff test direct 8 heures après application de chaque aérosol.

On évalue l'intensité de l'odeur de transpiration en donnant une note de 1 à 9 (1: intensité imperceptible et 9: intensité extrêmement forte). On évalue la valeur hédonique en donnant une note de 1 à 9 (1: odeur extrêmement désagréable et 9: extrêmement agréable)

| Aérosol testé | Intensité moyenne de l'odeur de transpiration | Valeur hédonique moyenne de l'odeur |
|---|---|---|
| **Exemple 2 (invention)** | 2,7 | 4,5 |
| **Exemple 3 (hors invention)** | 3,1 | 4,1 |
| **Pourcentage de variation** | -15% | +9% |

La composition de particules de gluconate de zinc selon l'invention à 0,9% en poids en matière active dans un aérosol alcoolique diminue significativement l'intensité de l'odeur de transpiration et le désagrément de l'odeur par rapport à l'aisselle traitée avec du Triclosan à 0,15% en poids en matière active dans le même support, 8 heures après application.

**ETUDE COMPARATIVE DU TEMPS DE DECANTATION DE l'ACTIF DEODORANT ET DE LA FACILITE A LA REDISPERSION DU GATEAU DANS UN AEROSOL ALCOOLIQUE APRES 8 JOURS DE REPOS**

On réalise les quatre aérosols alcooliques suivants :

| Composition | Exemple 2 (invention) | Exemple 5 (invention) | Exemple 6 (invention) | Exemple 7 (hors invention) |
|---|---|---|---|---|
| Composition de particules de gluconate de zinc selon l'exemple 1A | 45 | | | |
| Composition de particules de gluconate de zinc selon l'exemple 1B | | 6,57 | | |
| Composition de particules de gluconate de zinc selon l'exemple 3B | | | 6,75 | |

(suite)

| Composition | Exemple 2 (invention) | Exemple 5 (invention) | Exemple 6 (invention) | Exemple 7 (hors invention) |
|---|---|---|---|---|
| Particules de gluconate de zinc non- aciculaire (Givobio G Zn-Seppic) | - | | | 1 |
| Alcool éthylique absolu dénaturé | | 38,4 | 38,25 | |
| Alcool éthylique à 95°vol dénaturé | | | | 45 |
| Isobutane | 55 | 55 | 55 | 55 |

Préparation de l'exemple 5 et 6 :

La composition selon 1 B ou 3B selon l'invention est introduite dans un bécher contenant l'alcool. Le gluconate de zinc est dispersé au turax à une vitesse de 10 000 tours /minutes jusqu'à homogénéisation du mélange. La préparation est ensuite conditionnée en aérosol et pressurisée selon une méthode classique.

Préparation de l'exemple 7 :

Le gluconate est introduit dans l'alcool à 95° puis mélangé 5 minutes. Le temps est suffisamment court pour éviter la transformation des particules de gluconate en aiguilles. La préparation est ensuite conditionnée en aérosol et pressurisée par une méthode classique.
Les formules sont fabriquées dans des aérosols en verre de 100mL et sont remplis au tiers (soit 33,3 gr).

**Protocole du test du temps de décantation**

Un repère est fait au marqueur au niveau du liquide dans l'aérosol en verre pressurisé ainsi que 1 cm en-dessous de ce niveau. Après 24 heures au repos, l'aérosol est fortement agité, le temps de décantation est défini lorsque le front de décantation passe le repère à 1 cm.

**Protocole du test de facilité de redispersion du gâteau**

Après 8 jours laissé au repos sans agitation, la facilité à disperser le gâteau qui se trouve au fond de l'aérosol est mesurée par une agitation manuelle. La notation pour quantifier la facilité à disperser le gâteau est faites de façon subjective.
Très facile : dispersion sans grande agitation.
Facile : dispersion après une 1 à 2 agitations fortes. (Utilisation normal du produit)
Légèrement difficile : dispersion après une 3 à 4 agitations fortes
Difficile: dispersion après 5 à 7 agitations fortes.
Très difficile : Dispersion après 7 à 10 agitations fortes. Certains morceaux ne sont pas dispersés.
Impossible : Après 10 coups de fortes agitations, le gâteau reste collé au fond.

| Propriété mesurée | Exemple 2 5 (invention) | Exemple 5 (invention) | Exemple 6 (invention) | Exemple 7 (hors invention) |
|---|---|---|---|---|
| Temps de décantation à 24 heures (en secondes) | 60 | Aucune décantation après plusieurs semaines | Aucune décantation après plusieurs semaines | 10 |
| Dispersion du gâteau à 8 jours | Facile | Pas de gateau | Pas de gateau | Impossible |

## EXEMPLE 8 : SPRAY ALCOOLIQUE :

| Composition | | Quantités en % en poids |
|---|---|---|
| Phase A | Composition de particules de gluconate selon l'exemple 1A | 97,5 |
| Phase B | 2-éthylhexyl éther de glycérol | 1 |
| | Phénoxyéthanol | 0,5 |
| | Parfum | 1 |

On introduit la phase B dans la phase A constituée de la composition de l'exemple 1A. Le mélange est homogénéisé sous pales et raclantes.

## EXEMPLE 9 : STICK ANHYDRE :

| Composition | Quantités en % en poids |
|---|---|
| Aluminium chlorydrate | 20 |
| Composition de particules de gluconate selon l'exemple 2B | 1,5 |
| Cyclopentasiloxane (Dow Corning 245 Fluid) | 17 |
| PPG-14 butyl éther | 10 |
| Huile de ricin hydrogénée | 4 |
| Polydécène hydrogéné (Silkflo S366-Amoco) | 15 |
| Alcool stéarylique | 15 |
| PEG-8 Distéarate (Stéarinerie Dubois) | 2,5 |
| Benzoate d'alcools en $C_{12}$-$C_{15}$ (Finsolv TN-Witco) | 15 |

Préparation de l'exemple 9 :

Le gluconate de la composition 2B est filtré. Il est ensuite introduit lors de la prépartion du stick en même temps que l'aluminium chlohydrate. La dispersion du gluconate de zinc est effectuée au turax à une vitesse de 10 000 tours/minutes pendant 10 minutes. Le stick anhydre est formulé d'après un mode opératoire classique.

## EXEMPLE 10 : AEROSOL ANHYDRE :

| Composition | Quantités en % en poids |
|---|---|
| Aluminium chlorydrate | 7 |
| Composition de particules de gluconate selon l'exemple 2B | 1 |
| Citrate de triéthyle (Citroflex 2- Morflex) | 1 |
| Palmitate d'isopropyle | 1 |
| Benzoate d'alcools en $C_{12}$-$C_{15}$ (Finsolv TN-Witco) | 3 |
| Steralkonium Bentonite (Tixogel MP 250- Sud Chemie) | 0,5 |
| Cyclopentasiloxane (Dow Corning 245 Fluid) | 6,5 |
| Isobutane | 80 |

**Revendications**

**1.** Composition de particules de gluconate de zinc, **caractérisée par le fait qu'**elle comprend au moins des particules de gluconate de zinc sous forme aciculaire.

**2.** Composition selon la revendication 1, constituée d'un mélange de particules de gluconate de zinc non-aciculaire et de particules de gluconate de zinc sous forme aciculaire.

**3.** Composition selon la revendication 2 où les particules de gluconate de zinc sous forme non-aciculaire ont une taille moyenne de particule inférieure à 200 μm et de préférence allant de 5 à 165 μm et plus préférentiellement ayant une granulométrie moyenne pondérée en volume D (4,3) d'environ de 70 μm.

**4.** Composition selon la revendication 1, constituée exclusivement de particules de gluconate de zinc sous forme aciculaire.

**5.** Composition selon l'une quelconque des revendications 1 à 4, où les particules de gluconate de zinc sous forme aciculaire ont une longueur allant de 1 à 200 μm et un diamètre inférieur à 5μm.

**6.** Composition selon la revendication 5, où les particules de gluconate de zinc sous forme aciculaire ont une longueur allant de 5 à 50 μm et un diamètre est inférieur à 1 μm.

**7.** Composition selon l'une quelconque des revendications 1 à 6, **caractérisée par le fait qu'**elle se présente sous la forme d'une suspension dans un mélange de solvants comprenant au moins un solvant (A) solubilisant le gluconate de zinc et au moins un solvant (B) non-solubilisant le gluconate de zinc ; les proportions entre les solvants (A) et (B) étant telles que le gluconate de zinc est non-soluble dans le mélange de solvants (A) et (B) à température ambiante.

**8.** Composition selon la revendication 7, où le solvant (A) solubilisant est choisi parmi l'eau, la glycérine ou leurs mélanges

**9.** Composition selon la revendication 7, où le solvant (B) non-solubilisant est choisi parmi les monoalcools en $C_1$-$C_4$ ; les mono- ou polyalkylène glycols ou leurs éthers ainsi que leurs mélanges.

**10.** Procédé de préparation d'une composition de particules de gluconate selon l'une quelconque des revendications précédentes comprenant les étapes suivantes :

(a) un gluconate de zinc sous forme de poudre non aciculaire est mis en contact sous forte agitation avec au moins un solvant (A) solubilisant du gluconate de zinc et au moins un solvant (B) non-solubilisant dudit gluconate de zinc ; les proportions entre les solvants (A) et (B) étant telles que le gluconate de zinc est non-soluble dans le mélange de solvants (A) et (B).
(b) le mélange résultant est mis sous agitation jusqu'à formation de cristaux de gluconate de zinc en aiguilles et peut être chaufféjusqu'à 50°C pour accélérer la transformation du gluconate de zinc en particules aciculaires.
(c) éventuellement, après formation des aiguilles, on effectue ensuite une élimination des solvants par filtration ou évaporation.

**11.** Procédé selon la revendication 10, où le solvant (A) solubilisant est choisi parmi l'eau, la glycérine ou leurs mélanges

**12.** Procédé selon la revendication 10, où le solvant (B) non-solubilisant est choisi parmi les monoalcools à chaîne courte en $C_1$-$C_4$ ; les mono- ou polyalkylène glycols ou leurs éthers ainsi que leurs mélanges.

**13.** Composition de particules de gluconate de zinc susceptible d'être obtenue par le procédé de préparation tel que défini selon l'une quelconque des revendications 10 à 12.

**14.** Procédé de préparation d'une composition de particules de gluconate comprenant les étapes suivantes :

(a) on solubilise un gluconate de zinc sous forme de poudre non aciculaire dans au moins un solvant (A) solubilisant ledit gluconate de zinc ;

(b) on chauffe la solution à une température T supérieure ou égale à 40 °C ;

(c) on introduit au moins un solvant (B) ne solubilisant pas le gluconate de zinc ; les proportions entre les solvants (A) et (B) étant telles que le gluconate de zinc est soluble à la température T dans le mélange de solvants (A) et (B) et insoluble dans le même mélange à température ambiante ;

(d) le mélange résultant est refroidi à une température inférieure ou égale à la température ambiante sous agitation,

(e) éventuellement, après formation des aiguilles, on effectue ensuite une élimination des solvants par filtration ou évaporation.

**15.** Procédé selon la revendication 14, où le solvant (A) solubilisant est choisi parmi l'eau, la glycérine ou leurs mélanges

**16.** Procédé selon la revendication 14, où le solvant (B) non-solubilisant est choisi parmi les monoalcools à chaîne courte en $C_1$-$C_4$ ; les mono- ou polyalkylène glycols ou leurs éthers ainsi que leurs mélanges.

**17.** Composition de particules de gluconate de zinc susceptible d'être obtenue par le procédé de préparation tel que défini selon l'une quelconque des revendications 14 à 16.

**18.** Formulation cosmétique déodorante comprenant dans un support cosmétiquement acceptable au moins une composition de particules de gluconate de zinc telle que définie selon l'une quelconques des revendications 1 à 9, 13 et 17.

**19.** Formulation déodorante selon la revendication 18, dans laquelle la composition de particules de gluconate de zinc est présente dans des quantités allant de 0,01 à 10% en poids et plus préférentiellement de 0,1 à 5% en poids par rapport au poids total de la formulation.

**20.** Formulation déodorante selon la revendication 18 ou 19, sous forme de lotions, de crèmes ou de gels fluides distribués en spray aérosol, en flacon pompe ou en roll-on, sous forme de crèmes épaisses distribuées en tubes ou en grille ; sous forme de bâtonnets (sticks).

**21.** Formulation déodorante selon l'une quelconque des revendications 18 à 20, comprenant au moins une phase aqueuse.

**22.** Formulation selon la revendication 21, se présentant sous forme de lotion ou d'émulsion eau-dans-huile, huile-dans-eau, ou en émulsion.

**23.** Formulation selon la revendication 21, où la phase aqueuse contient de l'eau et un ou plusieurs solvants solubles ou miscibles dans l'eau.

**24.** Formulation selon la revendication 23, où les solvants solubles ou miscibles dans l'eau sont choisis parmi les solvants (B) tels que définis dans les revendications précédentes ou leurs mélanges avec des solvants (A) définis dans les revendications précédentes.

**25.** Formulation déodorante selon l'une quelconque des revendications 18 à 20, **caractérisée par le fait qu'**elle est anhydre.

**26.** Formulation selon l'une quelconque des revendications 18 à 25, **caractérisée par le fait qu'**elle comprend au moins une phase liquide organique non-miscible dans l'eau.

**27.** Formulation selon la revendication 26, dans laquelle la phase liquide organique comprend une ou plusieurs huiles émollientes siliconée ou hydrocarbonée, volatiles ou non-volatiles.

**28.** Formulation selon la revendication 27, dans laquelle les huiles émollientes sont présentes dans des quantités allant de 1 à 50% en poids et plus préférentiellement de 5 à 40% en poids par rapport au poids total de la composition.

**29.** Formulation selon l'une quelconque des revendications 26 à 28, **caractérisée par le fait qu'**elle comprend en plus un ou plusieurs agents structurants ou gélifiants de la phase liquide organique non-miscible dans l'eau.

**29.** Formulation selon l'une quelconque des revendications 18 à 29, contenant en plus un ou plusieurs actifs déodorants additionnels.

**30.** Formulation selon revendication 29, où les actifs déodorants additionnels sont choisis parmi les sels d'aluminium anti-transpirants.

**31.** Formulation selon la revendication 30, où les sels anti-transpirants sont choisis parmi les halohydrates d'aluminium ; les halohydrates d'aluminium et de zirconium ; les complexes d'hydroxychlorure de zirconium et d'hydroxychlorure d'aluminium avec un acide aminé.

**32.** Formulation selon la revendication 31, où le sel d'aluminium anti-transpirant est choisi parmi le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

**33.** Formulation selon la revendication 31, où le sel d'aluminium anti-transpirant est choisi parmi l'aluminium zirconium octachlorohydrate, l'aluminium zirconium pentachlorohydrate, l'aluminium zirconium tetrachlorohydrate, l'aluminium zirconium trichlorohydrate.

**34.** Formulation selon la revendication 31, où le sel d'aluminium anti-transpirant est choisi parmi les complexes d'hydroxychlorure de zirconium et d'hydroxychlorure d'aluminium avec la glycine.

**35.** Formulation selon la revendication 34, où le sel d'aluminium anti-transpirant est choisi parmi les complexes aluminium zirconium octachlorohydrex glycine, aluminium zirconium pentachlorohydrex glycine, aluminium zirconium tetrachlorohydrex glycine et aluminium zirconium trichlorohydrex glycine.

**36.** Formulation selon l'une quelconque des revendications 30 à 35, où le ou les sels d'aluminium anti-transpirants sont présents dans des quantités allant de 0,5 à 25% en poids par rapport au poids total de la composition.

**37.** Composition selon la revendication 29, où les actifs déodorants additionnels sont choisis parmi agents bactériostatiques ou agents bactéricides.

**38.** Composition selon l'une quelconque des revendications 18 à 37, **caractérisée par le fait qu'**elle comprend en plus un ou plusieurs agents de suspension.

**39.** Composition selon l'une quelconque des revendications 18 à 38, **caractérisée par le fait qu'**elle comprend en plus au moins une poudre organique.

**40.** Formulation selon l'une quelconque des revendications 18 à 39, **caractérisée par le fait qu'**elle comprend en plus au moins un additif cosmétique choisi parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs.

**41.** Formulation déodorante selon l'une des revendications 18 à 40, **caractérisée par le fait qu'**elle contient au moins un mono-alcool en $C_1$-$C_4$ et au moins un agent propulseur.

**42.** Dispositif aérosol constitué par :

    (A) un récipient comprenant une formulation cosmétique déodorante telle que définie selon l'une quelconque des revendications 18 à 41 ;
    (B) au moins un agent propulseur et un moyen de distribution de la dite composition aérosol.

**43.** Procédé cosmétique pour traiter les odeurs axillaires humaines, consistant à appliquer sur la surface axillaire une quantité efficace d'une composition de particules de gluconate de zinc telle que définie dans les revendications 1 à 9, 13 et 17. ou d'une formulation déodorante la contenant telle que définie dans les revendications 18 à 41.

**44.** Utilisation d'une composition de particules de gluconate de zinc telle que définie 1 à 9, 13 et 17 comme actif déodorant dans une composition cosmétique.

**Composition de gluconate de zinc nonaciculaire**

Fig1

**Composition 1B**

Fig2

**Composition 2B**

Fig3

**Composition 3B**

Fig4

**Office européen des brevets**

# RAPPORT DE RECHERCHE EUROPEENNE

Numéro de la demande

EP 05 29 0136

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | FR 1 181 752 A (KONINK IND MIJ VOORHEEN NOURY) 18 juin 1959 (1959-06-18) * le document en entier * ----- | 1,2 | A61K7/32 A61K7/48 |
| A,D | EP 0 768 080 A (OREAL) 16 avril 1997 (1997-04-16) * le document en entier * ----- | 1-45 | |
| A,D | WO 01/99376 A (HENKEL KGAA) 27 décembre 2001 (2001-12-27) * le document en entier * ----- | 1-45 | |
| A | DATABASE WPI Section Ch, Week 199909 Derwent Publications Ltd., London, GB; Class D22, AN 1999-099361 XP002293583 & JP 10 328284 A (LION CORP) 15 décembre 1998 (1998-12-15) * abrégé * ----- | 1-45 | |
| A | P.W. ROLLER ET AL.: "The Zinc Gluconate System" AUST. J. CHEM, vol. 29, 1976, pages 2395-2403, XP008048087 * le document en entier * ----- | 1-45 | **DOMAINES TECHNIQUES RECHERCHES (Int.Cl.7)** A61K |

Le présent rapport a été établi pour toutes les revendications

| Lieu de la recherche | Date d'achèvement de la recherche | Examinateur |
|---|---|---|
| La Haye | 8 juin 2005 | Fischer, J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 05 29 0136

La présente annexe indique les membres de la  famille de brevets relatifs aux documents  brevets cités dans le rapport de
recherche européenne visé ci-dessus.
Lesdits members sont contenus au fichier informatique de l'Office  européen des brevets à la date du
Les renseignements fournis sont donnés à titre  indicatif et n'engagent pas la responsabilité  de l'Office européen des brevets.

08-06-2005

| Document brevet cité au rapport de recherche | | Date de publication | Membre(s) de la famille de brevet(s) | | Date de publication |
|---|---|---|---|---|---|
| FR 1181752 | A | 18-06-1959 | AUCUN | | |
| EP 0768080 | A | 16-04-1997 | FR | 2739775 A1 | 18-04-1997 |
| | | | AT | 167627 T | 15-07-1998 |
| | | | BR | 9607243 A | 30-12-1997 |
| | | | CA | 2208243 A1 | 24-04-1997 |
| | | | CN | 1169111 A ,C | 31-12-1997 |
| | | | DE | 69600375 D1 | 30-07-1998 |
| | | | DE | 69600375 T2 | 29-10-1998 |
| | | | EP | 0768080 A1 | 16-04-1997 |
| | | | ES | 2120275 T3 | 16-10-1998 |
| | | | WO | 9714399 A1 | 24-04-1997 |
| | | | HU | 9702391 A2 | 28-05-1998 |
| | | | JP | 3006892 B2 | 07-02-2000 |
| | | | JP | 10500707 T | 20-01-1998 |
| | | | KR | 230830 B1 | 15-11-1999 |
| | | | PL | 320421 A1 | 29-09-1997 |
| | | | RU | 2141309 C1 | 20-11-1999 |
| | | | US | 6346238 B1 | 12-02-2002 |
| | | | US | 2002127193 A1 | 12-09-2002 |
| WO 0199376 | A | 27-12-2001 | DE | 10137901 A1 | 05-12-2002 |
| | | | AU | 9554001 A | 02-01-2002 |
| | | | WO | 0199376 A2 | 27-12-2001 |
| | | | EP | 1393518 A2 | 03-03-2004 |
| | | | PL | 364079 A1 | 13-12-2004 |
| JP 10328284 | A | 15-12-1998 | AUCUN | | |

EPO FORM P0460

Pour tout renseignement concernant cette annexe :  voir Journal Officiel de l'Office européen des  brevets, No.12/82